Europäisches Patentamt

European Patent Office

Office européen des brevets

(19)

(11) Numéro de publication : **0 235 187 B1**

(12) **FASCICULE DE BREVET EUROPEEN**

(45) Date de publication du fascicule du brevet :
**11.12.91 Bulletin 91/50**

(21) Numéro de dépôt : **86904851.2**

(22) Date de dépôt : **22.08.86**

(86) Numéro de dépôt international :
**PCT/FR86/00288**

(87) Numéro de publication internationale :
**WO 87/01375 12.03.87 Gazette 87/06**

(51) Int. Cl.$^5$ : **C07K 15/00, A61K 39/12,
C12P 21/00, A61K 39/42,
G01N 33/569, C12N 15/00,
C12N 15/37**

(54) POLYPEPTIDES ET ANTICORPS CARACTERISTIQUES DU PAPILLOMAVIRUS, METHODES DE DIAGNOSTIC ET VACCINS LES UTILISANT.

(30) Priorité : **26.08.85 FR 8512750**

(43) Date de publication de la demande :
**09.09.87 Bulletin 87/37**

(45) Mention de la délivrance du brevet :
**11.12.91 Bulletin 91/50**

(84) Etats contractants désignés :
**AT BE CH DE FR GB IT LI LU NL SE**

(56) Documents cités :
**EP-A- 0 092 456
EP-A- 0 133 123
EP-A- 0 192 001
Journal of Virology, vol. 52, no. 3, décembre
1984 American Soc. for Microbiology(US)
D.Kremsdorf et al.: "Molecular cloning and
characterization of the genomes of nine newly
recognized human papillomavirus types associated with epidermodysplasia verruciformis", pp 1013-1018, voir l'article en entier.
Nature, vol. 321, 15 Mai 1986, McMillan Journals Ltd, Basingstoke(GB) S.Beaudenon et al.:
"A novel type of human papillomavirus associated with genital neplasias",pp 246-249, voir
p 246, colonne de droite , lignes 1-16;page 247,
légende de la fig. 1, 'methods'.
J. Gen. Virol., vol. 65(GB) A.Roseto et
al.:"Monoclonal antibodies to the major capsid protein of human papillomavirus type I",
pp1319-1324, voir p. 1319, résumé en entier, p
1323, lignes 6-17**

(56) Documents cités :
**Chemical Abstracts, vol. 97, 1982, Columbus,
Ohio, US D. Kremsdorf et al.:"Biochemical
characterization of two types of human papillomaviruses associated with epidermodysplasia verruciformis", voir p. 161, résumé
121331v.
Journal of Virology, vol 36, no. 2, Nov 1980.
American Soc. for Microbiology(US) C A Heilman et al.: "Cloning of human papillomavirus
genomic DNAs and analysis of homologous
polynucleotide sequences", pp 395-407.**

(73) Titulaire : **INSTITUT PASTEUR
25/28, rue du Docteur Roux
F-75015 Paris (FR)**
Titulaire : **INSTITUT NATIONAL DE LA SANTE
ET DE LA RECHERCHE MEDICALE (INSERM)
101, rue de Tolbiac
F-75013 Paris (FR)**

(72) Inventeur : **KOMLY, Carol, Ann
6, rue Copreau
F-75015 Paris (FR)**
Inventeur : **CROISSANT, Odile
19, rue A. Lançon
F-75013 Paris (FR)**
Inventeur : **BREITBURD, Françoise
36, rue Molitor
F-75016 Paris (FR)**

(74) Mandataire : **Gutmann, Ernest et al
S.C. Ernest Gutmann - Yves Plasseraud 67,
boulevard Haussmann
F-75008 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 235 187 B1

## Description

L'invention concerne des produits d'expression de gènes contenus dans des ADNs de papillomavirus, ainsi que des procédés les mettant en oeuvre pour le diagnostic in vitro d'infections à papillomavirus. Elle concerne également des anticorps fournis contre les susdits produits d'expression.

Plus particulièrement, l'invention fournit des techniques perfectionnées d'identification de papillomavirus, par exemple obtenus à partir de lésions ou de coupes de biopsies et permet de faire des diagnostics améliorés quant à l'évolution possible des lésions en cause.

L'expression "papillomavirus" recouvre un grand nombre de virus ayant en commun d'être tenus pour responsables de plusieurs formes d'infections virales s'étageant entre les verrues cutanées ou de muqueuses relativement bénignes et des hyperplasies susceptibles de dégénérer en néoplasies intra-épithéliales et cancers cutanés. Parmi les infections à papillomavirus, on mentionnera également plus particulièrement les épidermodysplasies verruciformes, qui seront quelquefois ci-après désignées sous l'expression "EV".

Un certain nombre de types de papillomavirus a déjà été décrit, voir notamment J. Virol (Déc. 1984), pages 1013-1018. D'autres ont été identifiées dans la demande de brevet européen 192.001. Dans la présente demande de brevet il est fait référence à de nombreux types et sous-types de papillomavirus, parmi lesquels les papillomavirus décrits dans les documents identifiés ci-dessus. Ils ont été isolés à partir de verrues ou lésions maculaires disséminées et sont susceptibles de donner lieu au développement précoce de cancers de la peau chez d'importantes proportions des malades qui en sont affectés.

Il est fait plus particulièrement référence aux papillomavirus, axquels correspondent les ADNs recombinants décrits dans la demande de brevet européen 192.001 et dont les désignations suivent. Ils ont respectivement été déposés le 30 novembre 1984 à la C.N.C.M. (Collection Nationale des Cultures de Micro-Organismes de l'INSTITUT PASTEUR de Paris), sous les numéros apparaissant ci-après :

```
pBR322/HPV2d  .................... n° I-379
pBR322/HPV10bA................... n° I-380
pBR322/HPV10bB................... n° I-381
pBR322/HPV14a.................... n° I-382
pBR322/HPV14b.................... n° I-383
pBR322/HPV15  .................... n° I-384
pBR322/HPV17a .................... n° I-385
pHPV5 HindIIIB/HPV17b ........... n° I-386
pBR322/HPV19  .................... n° I-387
pBR322/HPV20  .................... n° I-388
pBR322/HPV21  .................... n° I-389
pHPV5 HindIIIB/HPV22 ........... n° I-390
pBR322/HPV23  .................... n° I-391
pBR322/HPV24a .................... n° I-392
pBR322/HPV24b .................... n° I-393
pBR322/HPV28  .................... n° I-394
pBR322/HPV29  .................... n° I-395
pBR322/HPV31  .................... n° I-396
pSP64/HPV32   .................... n° I-397
pLI55/IP2     .................... n° I-450
pSP65/IP4     .................... n° I-449
```

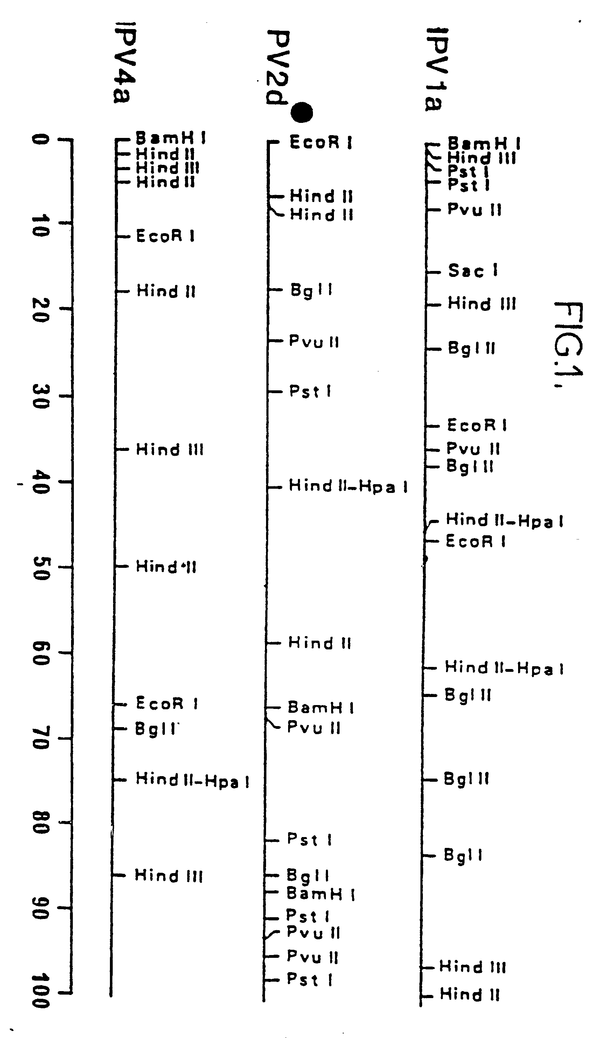

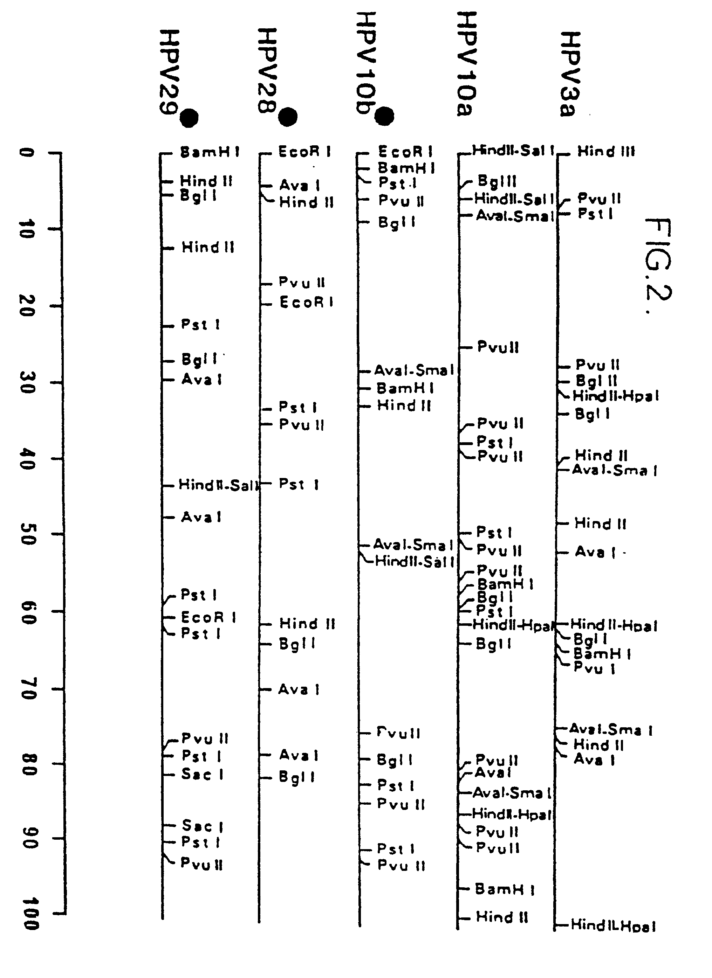

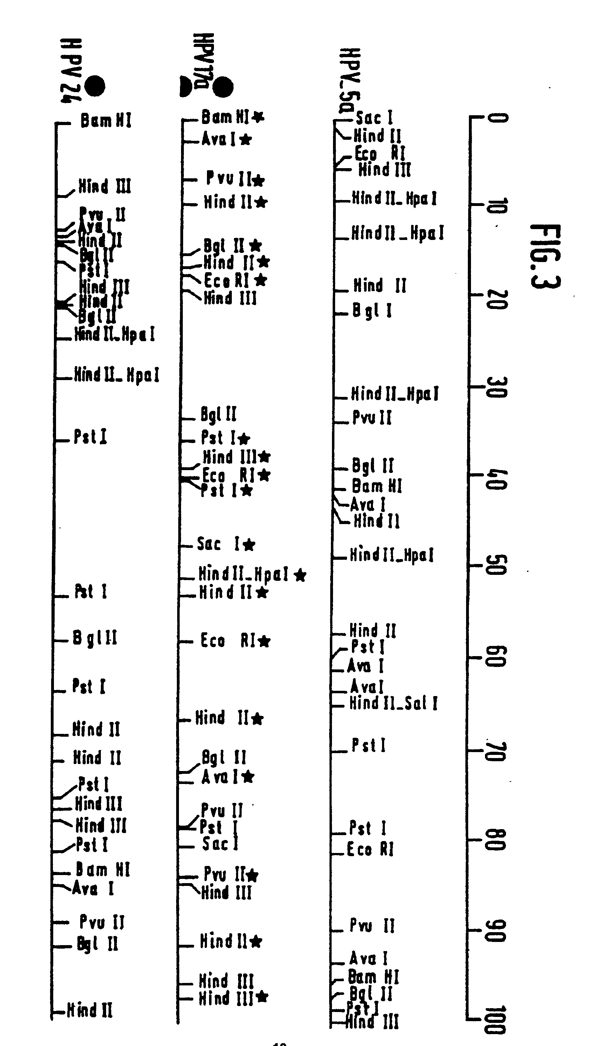

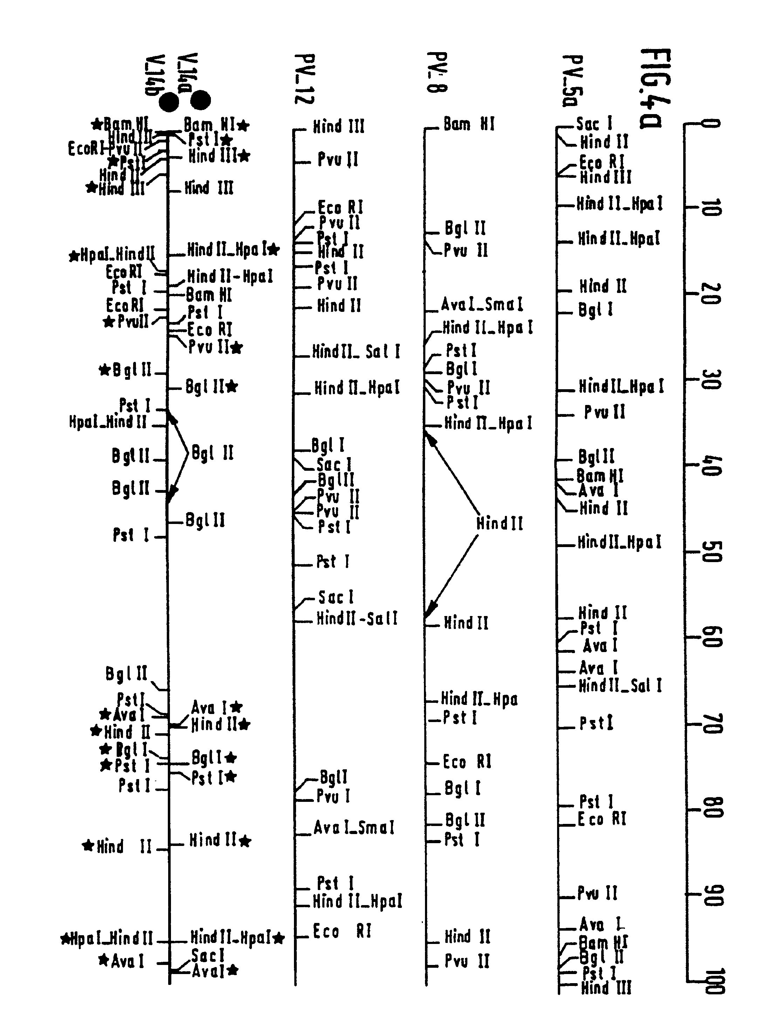

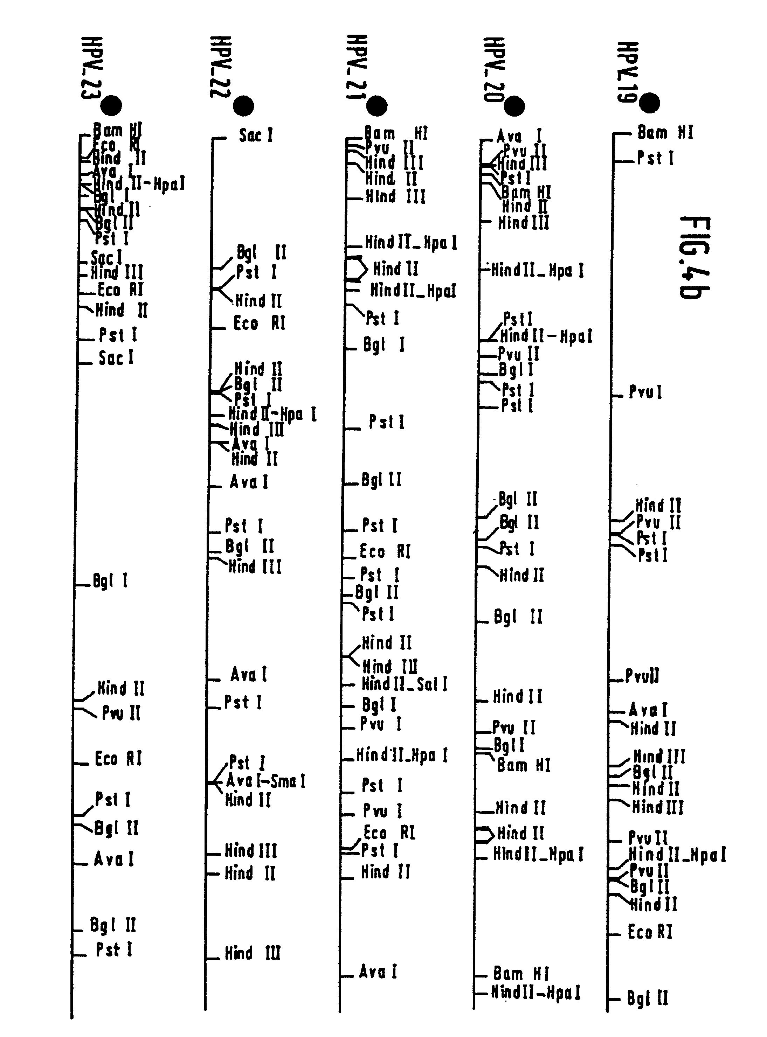

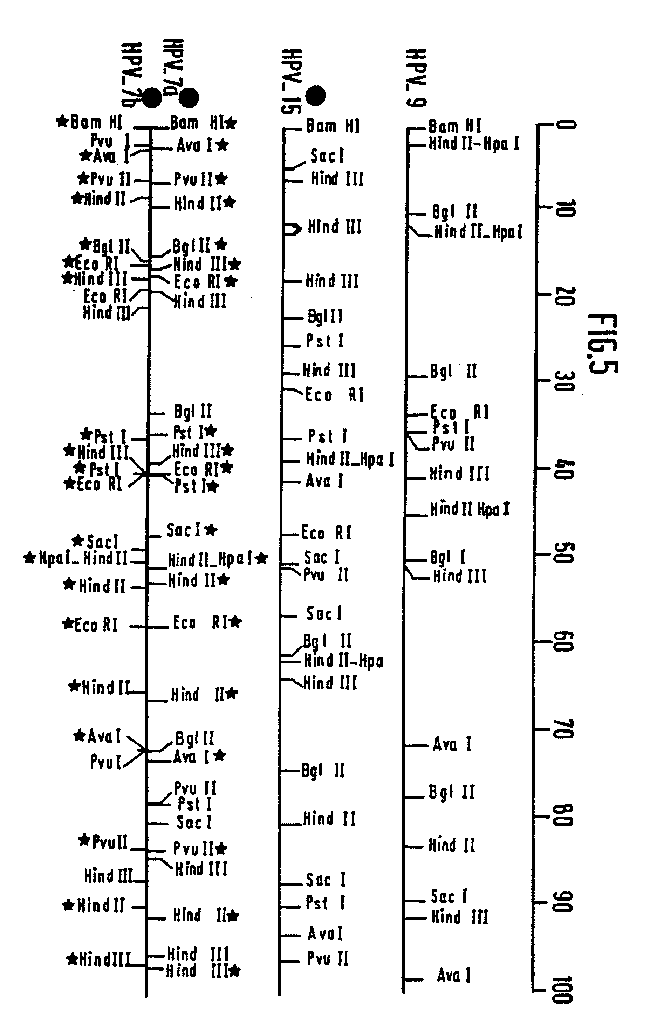

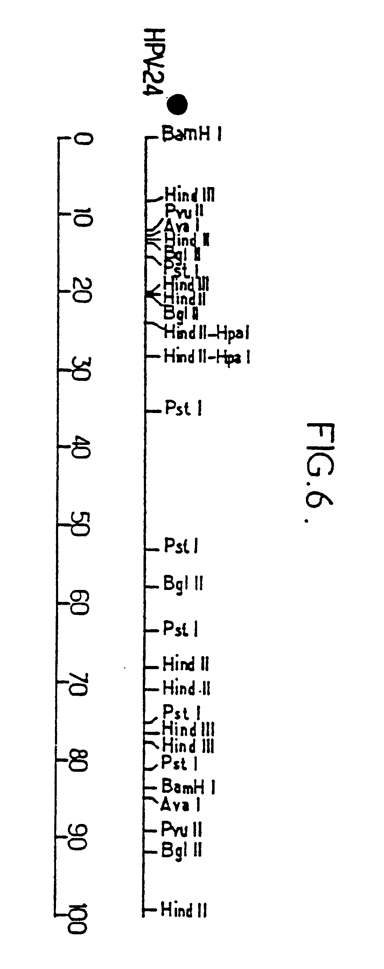

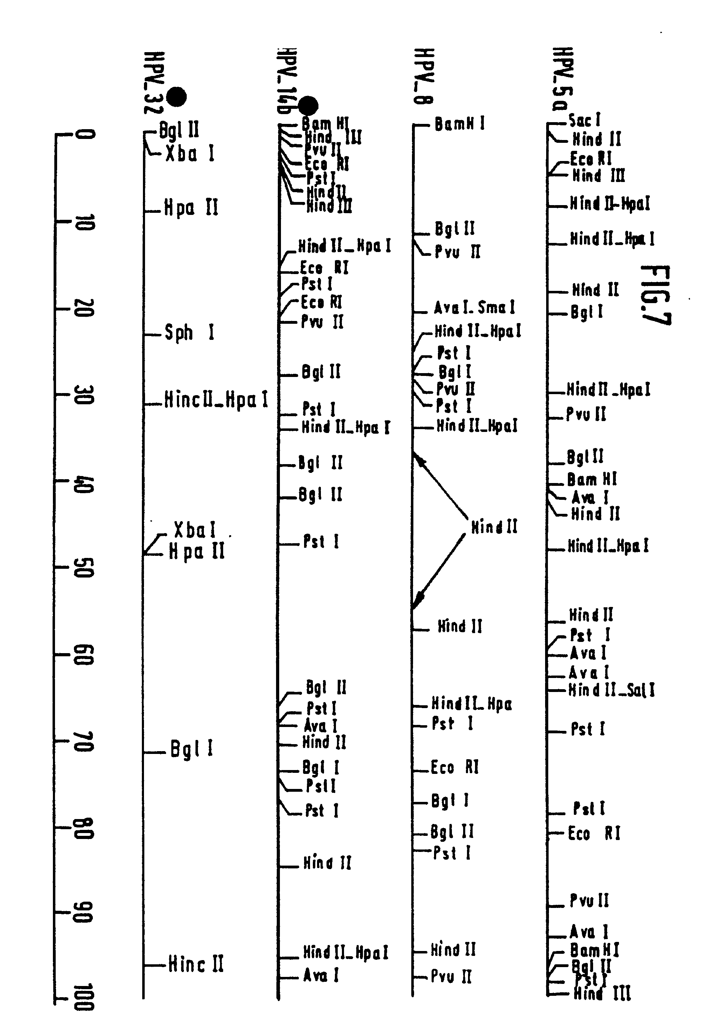

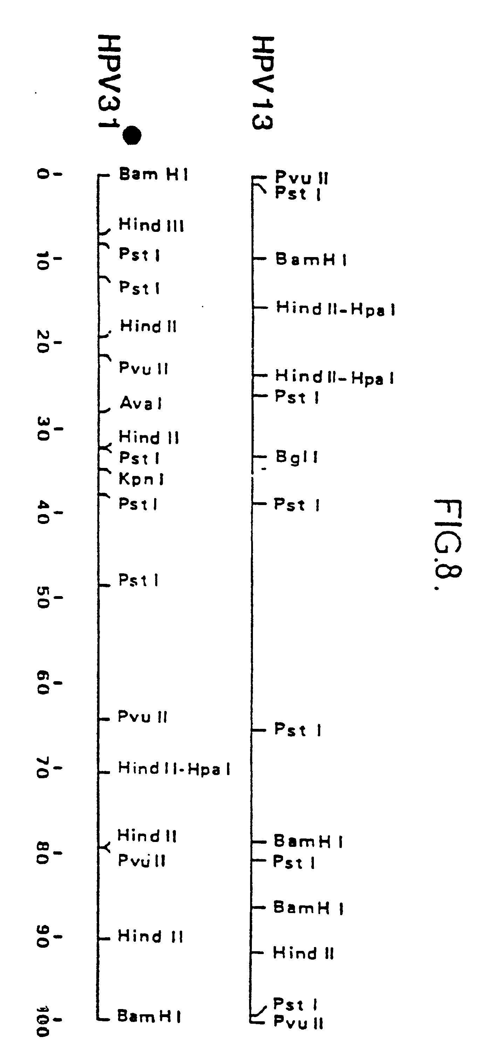

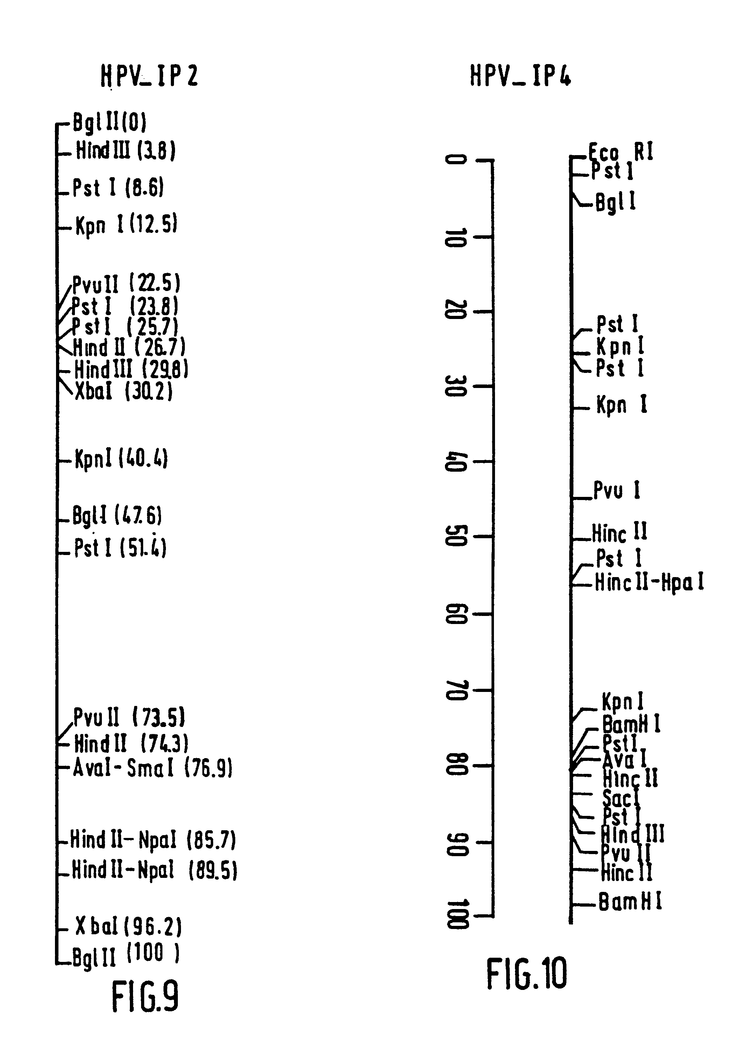

Les cartes de restriction de ces ADN-HPVs, et d'autres ADN-HPVs sont contenues dans les figures 1 à 10.

La demande de brevet européen 192.001 a également fait référence à des fragments d'ADN, issus des ADN-HPVs sus-décrits, et plus particulièrement à ceux correspondant respectivement aux gènes E6-E7 ; E1 ; L2 ; L1 et à leurs régions intergéniques. Les positions et longeurs relatives de ces divers fragments, vis-à-vis des sites pris comme origines dans les figures sont indiquées dans le tableau qui suit.

TABLEAU IV

LOCALISATION PUTATIVE DES PRINCIPAUX GENES ET DE LA REGION INTERGENIQUE SUR LES CARTES PHYSIQUES DES GENOMES D'HPV

| Type d'HPV | Coordonnées des extrémités 5' et 3' des fragments correspondant aux gènes | | | | à la région |
|---|---|---|---|---|---|
| | E6 - E7 | E1 | L2 | L1 | intergénique |
| 1 | 44 - 34,5 | 35 - 11 | 95 - 75,5 | 76,5 - 56 | 56 - 44,5 |
| 3 | 18,5 - 9 | 9,5 - 85,5 | 69.5 - 50 | 51 - 30,5 | 30,5 - 19 |
| 5 | 6,5 - 97 | 97,5 - 73,5 | 57,5 - 38 | 39 - 18,5 | 18,5 - 7 |
| 8 | 63 - 53,5 | 54 - 30 | 14 - 94,5 | 95,5 - 75 | 75 - 63,5 |
| 9 | 42 - 32,5 | 33 - 9 | 93 - 73,5 | 74,5 - 54 | 54 - 42,5 |
| 10a | 49 - 39,5 | 40 - 16 | 0 - 80,5 | 81,5 - 61 | 61 - 49,5 |
| 10b | 93 - 83,5 | 84 - 60 | 44 - 24,5 | 25,5 - 5 | 5 - 93,5 |
| 12 | 23,5 - 14 | 14,5 - 90,5 | 74,5 - 55 | 56 - 35,5 | 35,5 - 24 |
| 14 | 8,5 - 99 | 99,5 - 75,5 | 59,5 - 40 | 41 - 20,5 | 20,5 - 9 |
| 15 | 39,5 - 30 | 30,5 - 6,5 | 90,5 - 71 | 72 - 51,5 | 51,5 - 40 |
| 17 | 46 - 36,5 | 37 - 13 | 97 - 77,5 | 78,5 - 58 | 58 - 46,5 |
| 24 | 24,5 - 15 | 15,5 - 91,5 | 75,5 - 56 | 57 - 36,5 | 36,5 - 25 |
| 28 | 47,5 - 38 | 38,5 - 14,5 | 98,5 - 79 | 80 - 59,5 | 59,5 - 48 |
| 29 | 89,5 - 80 | 80,5 - 56,5 | 40,5 - 21 | 22 - 1,5 | 1,5 - 90 |
| 31 | 89 - 78,5 | 80 - 53,5 | 33,5 - 15,5 | 17,5 - 96,5 | 96,5 - 92,5 |

EP 0 235 187 B1

L'invention concerne encore plus particulièrement les produits d'expression des gènes E6, E7 et surtout L2, des différents papillomavirus qui ont été évoqués dans ce qui précède.

Ces produits d'expression peuvent eux-mêmes être utilisés pour la détection de papillomavirus ou de leurs produits d'expression dans des prélèvements biologiques déterminés et pour l'identification des papillomavirus selon les types ou sous-types auxquels ils peuvent appartenir. Les conditions dans lesquelles ces produuits d'expression peuvent être obtenus seront illustrées dans la suite de cette description, notamment en rapport avec l'expression de la séquence L2 du papillomavirus HPV 1.a, étant entendu que des techniques semblables peuvent être utilisées pour induire l'expression des séquences génétiques L2 (ou de E6, E7 ou L1) provenant d'autres types de papillomavirus. Dans ce qui suit, ils sera, sauf exception, plus particulièrement fait référence aux séquences ou gènes L2, étant entendu cependant que les enseignements fournis à propos des produits d'expression des gènes L2 peuvent éventuellement être transposés aux produits d'expression des autres gènes dont question ci-dessus. Les produits d'expression des séquences L2 présentent cependant un intérêt tout particulier, en ce qu'ils peuvent eux-mêmes être utilisés pour la production in vivo d'anticorps susceptibles de reconnaître les produits d'expression du gène L2 dans des prélèvements biologiques affectés par le papillomavirus correspondant et pas par un papillomavirus de type différent, et plus particulièrement lorsque les préparations du genre en question ont préalablement été fixées.

L'invention concerne en particulier un ensemble de compositions, dont chacune est formée d'un groupe comprenant au moins un polypeptide, chaque polypeptide correspondant au produit d'expression de tout ou partie d'un gène E6, E7 ou, de préférence, L2, d'un papillomavirus, ces groupes étant respectivement dérivés des papillomavirus suivants :

1) HPV 2d (C.N.C.M. N° I-379) ;
2) HPV 10b (C.N.C.M. N° I-380 et N° I-381), HPV 28 (C.N.C.M. N° I-395) ;
3) HPV 24 (C.N.C.M. N° I-392 et I-393) ;
4) HPV 14 (C.N.C.M. N° I-382 et I-383), HPV 15 (C.N.C.M. N° I-384), HPV 17 (C.N.C.M. N° I-385 et N° I-386), HPV 19 (C.N.C.M. N° I-387), HPV 20 (C.N.C.M. N° I-388), HPV 21 (C.N.C.M. N° I-389), HPV 22 (C.N.C.M N° I-390) et HPV 23 (C.N.C.M. I-391) ;
5) HPV 15 (C.N.C.M. N° I-384), HPV 17 (C.N.C.M. N° I-385 et N° I-386) ;
6) HPV 24 (C.N.C.M. N° I-392 et N° I-393) ;
7) HPV 14 (C.N.C.M. N° I-382 et N° I-383), HPV 32 C.N.C.M. N° I-397) et HPV IP4 (C.N.C.M. N° I-449) ;
8) HPV 31 (C.N.C.M. N° I-396) ;
9) HPV 32 (C.N.C.M. N° I-397) ;
10) HPV 16, HPV 18 et HPV IP2 (C.N.C.M. N° I-450), étant entendu que les polypeptides des dix compositions sont respectivement choisis de façon à être en toutes circonstances différents les uns des autres, dans la mesure où chacune des dix compositions serait réduite à un polypeptide dérivé de l'un seulement des HPVS du groupe correspondant.

L'invention fournit aussi des moyens distincts permettant de déterminer les types de papillomavirus présents dans une lésion étudiée, afin d'en évaluer la gravité et de choisir la thérapie à suivre. Les anticorps produits contre les différents types de produits d'expression des gènes L2 peuvent être groupés en mélanges correspondant à ceux qui ont été identifiés plus haut en rapport avec les polypeptides.

L'invention fornit par conséquent ausssi des nécessaires ou "kits" comportant une pluralité d'anticorps distincts et permettant des essais successifs de détection, le cas échéant d'identification ou de classification, de papillomavirus nouvellement isolés. Un nécessaire ou kit conforme à l'invention comprend un ensemble de compositions d'anticorps, dont chacune est formée d'un groupe comprenant au moins un anticorps lui-même dirigé contre un produit d'expression de tout ou partie d'un gène E6, E7, ou, de préférence, L2, d'un papillomavirus, ces groupes étant respectivement dérivés des papillomavirus suivants :

1) HPV 2d (C.N.C.M. N° I-379) ;
2) HPV 10b (C.N.C.M. N° I-380 et N° I-381), HPV 28 (C.N.C.M. N° I-395) ;
3) HPV 24 (C.N.C.M. N° I-392 et I-393) ;
4) HPV 14 (C.N.C.M. N° I-382 et I-383), HPV 15 (C.N.C.M. N° I-384), HPV 17 (C.N.C.M. N° I-385 et N° I-386), HPV 19 (C.N.C.M. N° I-387), HPV 20 (C.N.C.M. N° I-388), HPV 21 (C.N.C.M. N° I-389), HPV 22 (C.N.C.M N° I-390) et HPV 23 (C.N.C.M. I-391) ;
5) HPV 15 (C.N.C.M. N° I-384), HPV 17 (C.N.C.M. N° I-385 et N° I-386) ;
6) HPV 24 (C.N.C.M. N° I-392 et N° I-393) ;
7) HPV 14 (C.N.C.M. N° I-382 et N° I-383), HPV 32 (C.N.C.M. N° I-397) et HPV IP4 (C.N.C.M.N° I-449) ;
8) HPV 31 (C.N.C.M. N° I-396) ;
9) HPV 32 (C.N.C.M. N° I-397) ;
10) HPV 16, HPV 18 et HPV IP2 (C.N.C.M. N° I-450), étant entendu que les anticorps des dix compositions sont respectivement choisis de façon à être en toutes circonstances différents les uns des autres, dans la

5

mesure où chacune des dix compositions serait réduite à un anticorps dérivé de l'un seulement des anticorps du groupe correspondant.

Les anticorps formés contre les produits d'expression des gènes L2 (ou contre des protéines recombinantes contenant ces produits d'expression fusionnés avec un polypeptide supplémentaire, particulièrement un polypeptide stabilisant, n'entraînant pas la modification des propriétés immunogènes du produit d'expression des gènes L2) sont utilisables pour la détection directe de virus dans des coupes histopathologiques provenant de lésions induites par les papillomavirus chez les sujets quien sont affectés. Avantageusement, la détection se fait sur des préparations fixées au préalable dans des conditions dissociantes, par exemple avec le milieu ou mélange de CARNOY déjà mentionné plus haut (également décrit dans l'ouvrage de L. LISON, intitulé (Histochimie et cytochimie animales").

Les anticorps anti-L2 éventuellement fixés peuvent être reconnus par d'autres anticorps formés contre les premiers, ces autres anticorps portant des marqueurs appropriés, de préférence non radioactifs. Ces marqueurs sont par exemple de nature enzymatique ou fluorescente.

L'invention concerne naturellement également les polypeptides eux-mêmes qui résultent de l'expression des gènes L2 des papillomavirus. Ces produits d'expression ont déjà dans ce qui précède été dénommés "protéines L2". Elle concerne encore les polypeptides dans lesquels cette protéine L2 se trouve fusionnée à d'autres séquences polypeptidiques, dans la mesure où celles-ci ne modifient pas de façon essentielle les propriétés immunogènes de la protéine L2. La présence de ces autres fragments polypeptidiques peut notamment résulter du mode de production utilisé de ces polypeptides hybrides, surtout lorsqu'ils ont été obtenus par des procédés mettant en jeu des techniques du génie génétique. Avantageusement, l'invention concerne des polypeptides hybrides contenant une séquence dérivée de la bêta-galactosidase. De tels produits peuvent notamment être obtenus par transformation de E. coli avec des vecteurs appropriés (phages ou plasmides) modifiés par tout ou partie de l'opéron lactose et comportant en outre, insérée en aval du promoteur de l'opéron-lactose (ou de tout autre promoteur approprié, par exemple de phage lambda), la séquence nucléotidique dérivée d'un gène L2 issu d'un papillomavirus de type déterminé. On a avantageusement recours à des plasmides ou phages de ce type comprenant une partie au moins du gène de la bêta-galactosidase de l'opéron-lactose.

L'invention concerne également des groupes de polypeptides distincts, ces polypeptides correspondant chaque fois à une partie seulement des protéines L2 complète dont il a été question plus haut, étant cependant entendu que les polypeptides de ces divers groupes comprennent chaque fois les sites antigéniques caractéristiques des protéines L2 du genre en question.

Les polypeptides selon l'invention peuvent également, lorsqu'ils ont été purifiés, être mis en oeuvre dans des techniques de purification des anticorps qui leur correspondent, notamment à partir de sérums d'animaux qui avaient été immunisés par ces polypeptides. En particulier, ces polypeptides peuvent être fixés sur des colonnes d'affinité. Les opérations de purification des anticorps consistent alors à faire passer le sérum les contenant au contact de colonnes d'affinité portant les susdits polypeptides. Les anticorps sélectivement fixés sur ces colonnes peuvent ensuite être récupérés par dissociation des complexes antigènes-anticorps, au moyen d'un tampon approprié, présentant une force ionique adéquate, par exemple une solution d'un sel tel que l'acétate d'ammonium. On peut également avoir recours à des solutions acidifiées.

L'invention concerne enfin les compositions mettant en jeu ces antigènes (ou groupes d'antigènes) et anticorps (ou groupes d'anticorps).

En particulier, l'invention concerne des groupes contenant l'un ou, de préférence, un "cocktail" d'anticorps dérivés des ensembles de papillomavirus, tous réputés être souvent présents dans un type d'affection donné. Ces cocktails (contenant ces anticorps ou groupes d'anticorps : mélange de sérums ou compositions d'anticorps purifiés en association avec un véhicule pharmaceutique approprié) sont alors susceptibles d'être mis en oeuvre par administration, notamment parentérale, au malade concerné, dans le traitement de l'affection donnée, dès lors que celle-ci aura été diagnostiquée cliniquement, à l'issue d'un essai de diagnostic in vitro sur un prélèvement histologique ou cytologique provenant de ce malade, ou qu'un essai de diagnostic in vivo aura révélé l'appartenance du papillomavirus infectieux à un type semblable à celui de l'un des papillomavirus de l'ensemble sus-indiqué. Ces sérums sont alors susceptibles de provoquer une régression des infections induites par les papillomavirus de types ou sous-types correspondants.

L'invention concerne enfin les compositions vaccinantes correspondantes contenant une ou de préférence plusieurs protéines L2, en association avec un véhicule pharmaceutiquement acceptable adapté au mode d'administration choisi, notamment par voie parentérale utilisable pour protéger les personnes soumises à de hauts risques d'être atteints par l'affection correspondante.

Il est enfin fait référence aux articles dont les références bibliographiques suivent, lesquels complètent en tant que de besoin la description de l'état de la technique antérieure, dans la mesure où cela pourrait s'avérer utile à la compréhension complète du texte par le lecteur. A ce titre, le contenu de ces articles doit donc être considéré comme faisant partie de la description.

Une méthodologie préférée pour produire une protéine L2 (ou un fragment de cette protéine) mettant en jeu un fragment de phase ouverte du gène L2 d'un papillomavirus donné, va être illustrée en rapport avec la description de la construction d'un vecteur incorporant ces fragments de phase ouverte, puis des conditions dans lesquelles on peut induire l'expression de la protéine L2 ou de ce polypeptide dans E. coli. Un procédé de purification de la protéine ou de ce polypeptide ainsi que sa mise en oeuvre dans la production de sérums contenant des anticorps dirigés contre ces protéines ou ce polypeptide seront également décrits.

Il sera clair pour l'homme du métier que le fragment utilisé de phase ouverte de L2 devra chaque fois être fusionné en phase dans le vecteur utilisé, le cas échéant avec un gène codant pour une protéine assurant la stabilité ou facilitant la purification ultérieure de la protéine hybride alors formée.

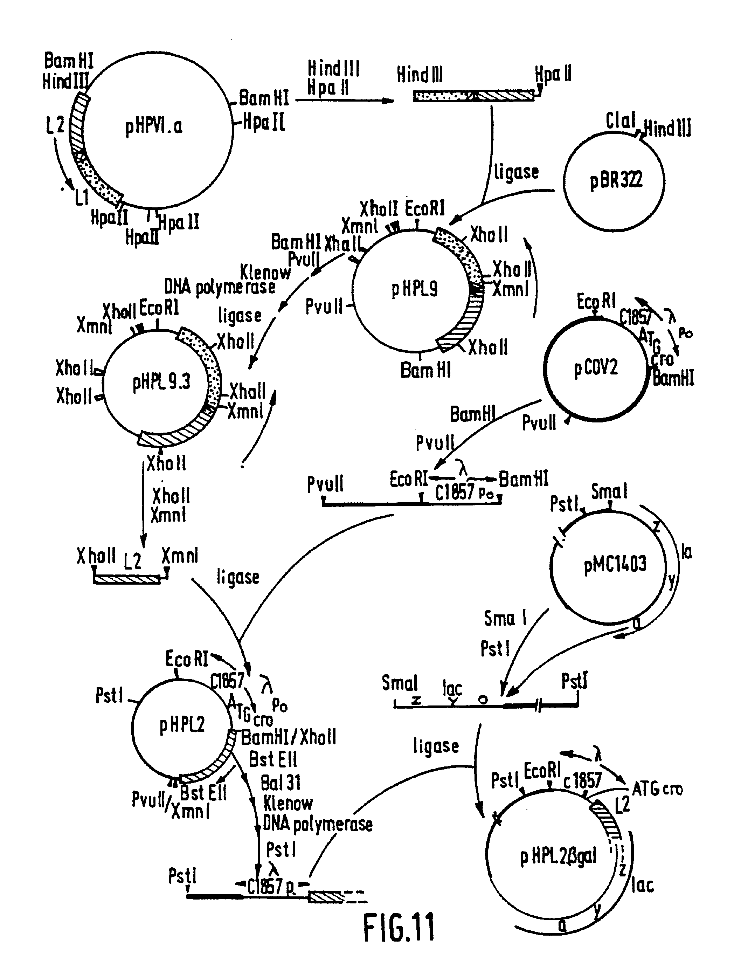

La séquence de fabrication est illustrée dans la figure 11 ci-jointe.

## MATERIELS ET METHODES.

### 1) TRAITEMENT DES ADNs.

En ce qui concerne les recombinaisons d'ADNs, la préparation des plasmides et des fragments d'ADN, la formation des extrémités franches, la digestion par l'enzyme Bal 31 et enfin la transfection des cellules, on a eu recours aux Techniques de Maniatis, T. et al. (1982), décrites dans le chapitre "Molecular cloning" (Clonage moléculaire) de l'ouvrage "A Laboratory Manuel" (Manuel de Laboratoire) de Cold Spring Harbor Laboratory, Cold Spring Harbor, NEW-YORK, U.S.A.

### 2) BACTERIES, PLASMIDES ET VIRUS.

Les ADNs plasmidiques ont été obtenus à partir de pHPVI.a (Danos et al, 1982 et brevet français 82 05887 du 5 Avril 1982) contenues dans E. coli (cellules C 600), de pCQV2 qu'hébergeaient des cellules RRI (queen, 1983) et de pMC1403 qu'hébergeaient des cellules MC1000 (Casadaban, 1983).

Les plasmides intermédiaires construits et le plasmide pHPL2 ont été passés plusieurs fois dans des cellules de E. coli MM 294 et les plasmides pHPL2-bêta-galactosidase dans des cellules MC1000 déficientes en béta-galactosidase. Les souches Lac$^+$ ont été détectées sous forme de colonies rouges sur des plaques d'agar de Mc Conkey contenant du lactose (Silhavy et al, 1984, dans "Experiments with gene fusions" - Expériences avec des fusions de gènes - : Cold Spring Harbor Laboratory, Cold Spring Harbor, NEW-YORK).

Les plasmides pHPL2 et pHPL2-bêta-gal ont ensuite été transfectés dans une souche lon⁻ déficiente en protéase, CAG1139 (Grossman et al, 1983, Cell 32, 151-159).

### 3) PREPARATION DE LYSATS BACTERIENS ET LEUR ANALYSE SUR GEL DE POLYACRYLAMIDE AVEC DODECYLSULFATE DE SODIUM (PAGE-SDS).

Après culture pendant une nuit, des cellules CAG 1139 contenant pHPL2 ou pHPL2-bêta-gal 116 ont été diluées de 5 à 20 fois dans un milieu LB. Elles ont été ensuite cultivées à 30°C, jusqu'à atteindre une Densité Optique 600 de 0,5-0,9.

Le promoteur lambda-PR a ensuite été déréprimé en faisant pousser les cellules à 41°C pendant 90 minutes. Les cultures ont été recentrifugées et les culots recueillis, suspendus dans un milieu 62 mM Tris, pH 6,8 contenant 2 % de SDS, 26 % de glycérol, du 2-mercaptoéthanol 2 M et 0,03 % de bleu de bromophénol. Un lysat de cellules CAG 1139 ne contenant pas de plasmide a été utilisé comme témoin.

Après traitement à 100°C pendant 10 minutes, des échantillons ont été soumis à électrophorèse dans un gel de polyacrylamide - SDS à 10 % selon la technique de Laemli (1970), Nature, 227, 680-684. Des protéines pré-colorées BRL de poids moléculaires connus étaient également soumises à électrophorèse.

Les bandes de protéines ont été visualisées par coloration avec le colorant connu sous la désignation "amido black" et transférées sur une feuille de nitro-cellulose pour analyse par la méthode dite "western blot" (transfert "Western").

### 4) ANALYSE.

L'analyse par "Western Blot" a été réalisée comme décrit par Rosetto et al (1984) J. Gen. Virol. 65, 1319-1324, en mettant en oeuvre des anticorps polyclonaux de papillomavirus (Orth et al (1978), Virology 91, 243-255 et Rosetto et al déjà cité) et des immunoglobulines IgG anti-cobaye et anti-lapins conjugués à de la peroxydase de Raifort.

## 5) ANALYSE D'ANTICORPS.

On a eu recours aux techniques d'immunodiffusion, d'immunofluorescence et d'immunoperoxydase antérieurement décrites (Orth et al, 1984, et Rosetto et al, 1984) en utilisant des particules virales de HPVI.a purifiées ou des coupes de lésions induites par des types de papillomavirus distincts.

## 6) PURIFICATION DES PROTEINES DE FUSION L2-BETA-GALACTOSIDASE.

Une culture de CAG 1139 hébergeant pHPL2-bêta-galactosidase 116 a été diluée 200 fois dans le milieu LB et a été cultivée jusqu'à atteindre une D.0.600 de 0,32. La culture a alors été rapidement portée à 41°C et cultivée à haute température pendant 90 minutes.

Le culot de cellules a été resuspendu dans un milieu Tris 20 mM, pH 7,5, Mg $Cl_2$ 10mM, et soumis à un traitement de rupture des cellules aux ultrasons.

La protéine de fusion soluble dans la préparation a été purifiée par chromatographie d'affinité sur une colonne p-aminophényl-β-D-thiogalactoside (TPEG)-SEPHAROSE comme décrit par Ullmann (1984). Gene 29, 27-31.

La protéine de fusion purifiée comme indiqué ci-dessus a été utilisée pour immuniser des cobayes Harley femelles. Les animaux reçurent 3 injections sous-cutanées, à des sites distincts, d'environ 150 microgrammes de protéines, en présence d'adjuvant complet de Freund (DIFCO) à des intervalles de 2 à 3 semaines. Le sérum a été prélevé une semaine après la troisième injection.

## RESULTATS.

## CONSTRUCTION DE PLASMIDES CAPABLES D'EXPRIMER LE CADRE DE LECTURE OUVERTE L2 DANS ESCHERICHIA COLI.

Les constructions sont représentées à la figure 1. Le cadre de lecture ouverte L2 a été cloné en vue de son expression dans Escherichia coli (E. coli). Le plasmide pHPVI.a contient le génome entier de HPVI.a cloné sur le site BamHI du plasmide pBR322 par Danos et al (EMBO J.I, p. 231-236, 1982). Dans le but d'isoler les cadres de lecture ouverte de la région tardive du génome viral, on a subcloné un fragment Hind III-Hpa II d'un plasmide pHpV I.a dans un plasmide pBR322. Le plasmide obtenu a été appelé pHPL9. Ce plasmide a été raccourci en enlevant un fragment BamHI-PVu II non essentiel dans la région pBR322. A partir du plasmide résultant pHPL 9.3, on a isolé un fragment Xholl-Xmnl comportant les 1526 paires debase (p.b.) du cadre de lecture ouverte moins les 318 paires terminales 5'.

Le codon d'arrêt du cadre de lecture ouvert L2 est conservé.

Le fragment L2 obtenu a ensuite été inséré dans le vecteur d'expression pCQV2 (queen 1983) à la place d'un fragment BamHI-PvuII non essentiel. Le cadre de lecture ouverte L2 se trouve ainsi directement adjacent au codon ATG de départ et à la séquence SD (Shine and Dalgarno, Proc. Natl. Acad. Sci. USA 71, p. 1342-1346, 1974) du gène cro du phage lambda et sous le contrôle du promoteur lambda PR. Le promoteur est régulé par le répresseur CI857 sensible à la température, lui permettant de contrôler la production du produit d'expression de L2. On a ainsi obtenu le plasmide pHPL2 dans lequel les 1206 paires de bases de la partie C terminale du cadre de lecture ouverte L2 sont en phase avec le codon de départ ATG de cro du phage lambda. Le respect de la phase de lecture a été vérifié par la présence d'un site de restriction Xholl à la jonction BamHI/Bgl II.

Dans le but de stabiliser la protéine L2 synthétisée dans E. coli et de disposer d'une méthode convenable pour la purifier, il a été décidé de l'exprimer aussi comme une protéine hybride fusionnée avec la bêta-galactosidase de E. coli.

Pour réaliser ceci, le codon final L2 a été éliminé après linéarisation du plasmide pHPL2 au site BstEII, suivi par une digestion avec l'enzyme Bal31.

Cet ADN, qui comporte le gène L2 et les séquences fournissant les signaux de transcription et de traduction, a été raccourci par digestion avec PstI, avant d'être inséré dans le plasmide pMC1403 (Casadaban, Methods in enzymology 100, p. 293-308, 1983) à la place du fragment pstI-SmaI.

PMC1403 comporte l'opéron lac sans son promoteur et les 22 premières paires de bases de la phase ouverte du gène de la bêta-galactosidase. Le recombinant a été transfecté dans les cellules MC 1000, lac⁻. La production à température élevée de bêta-galactosidase dans les clones obtenus a permis de sélectionner sur des plaques agar lactose Mc Conkey (Silhavez et al, Cold Spring Harbor Laboratory, N.Y. 1984) les plasmides pHPL2 bêta gal dans lesquels les gènes pour le L2 et le bêta-galactosidase sont en phase.

Le clone pHPL2, bêta gal 116 sélectionné selon une telle méthode a été étudié ultérieurement.

Le séquençage de l'ADN (résultats non montrés ici) a indiqué que seulement les deux derniers acides aminés C-terminaux du cadre de lecture ouvert L2 avaient été perdus au cours de la digestion par Bal 31 et que le produit exprimé par le cadre de lecture ouverte L2 était lié par une proline au neuvième acide aminé de la bêta-galactosidase.

## PRODUCTION DE PROTEINES APPARENTEES AU HPVI.a PAR DES CELLULES TRANSFECTEES AVEC pHPL2 et pHPL2.bêta gal 116.

Le plasmide pHPL2 a une capacité de codage pour une protéine L2 d'environ 51.2 Kd et de pHPL2-béta gal pour une protéine hybride d'environ 167 Kd.

Pour étudier la production de protéines par ces plasmides, ils ont d'abord été transfectés dans une souche de E. coli CAG 1139 lon⁻, protéase déficiente. Les cellules ont ensuite été cultivées à 30°C avant d'être exposées à 41°C, en vue d'induire la production de protéines sous le contrôle du promoteur lambda PR. Les lysats bactériens obtenus ont été électrophorisés sur un gel de polyacrylamide - SDS.

Les protéines apparentées aux virus HPVI.a ont été révélées par analyse selon la technique dite du "Western Blot", avec des anticorps anti-virus HPVI.a. Pour les cultures ayant poussé à 30°C, les résultats d'analyse ont été les mêmes pour les cellules contenant ou ne contenant pas un plasmide, mais pour les cellules induites à 41°C, des bandes spécifiques de protéines ont été démontrées dans les cellules transfectées : parmi les protéines isolées à partir des cellules transfectées avec le recombinant pHPL2-bêta gal 116, on a isolé une bande principale ayant le poids moléculaire attendu, d'approximativement 167 Kd, correspondant à une protéine de fusion L2 bêta-galactosidase (L2-bêta gal), et plusieurs bandes mineures de poids moléculaires d'environ 58 Kd, résultant probablement d'une dégradation protéolytique.

Pour la construction pHPL2, une bande de protéine d'environ 72 Kd a été révélée. Ce poids moléculaire est plus élevé que celui espéré de 51,2 Kd. La signification de cette différence reste à élucider.

Pour étudier ensuite l'antigénicité de la protéine produite dans E. coli, on a purifié le produit L2-bêta gal du lysat bactérien d'une culture induite par la chaleur, par affinité chromatographique sur une colonne de TPEG-SEPHAROSE (Ullman, Gene 29, p. 27-31, 1984). Les protéines éluées de la colonne ont été analysées sur un gel de polyacrylamide-SDS. La coloration avec un "amido-black" a révélé trois bandes de protéines principales, l'une de poids moléculaire élevé correspondant probablement à la protéine hybride L2-bêta gal, une seconde migrant conjointement avec la bêta-galactosidase prifiée et une troisième avec un poids moléculaire d'environ 60 Kd.

Cette protéine de bas poids moléculaire pourrait correspondre à un contaminant. L'analyse par la technique du "Western Blot" avec un anticorps spécifique du type HPVI.a a révélé une bande principale de poids moléculaires élevé correspondant au produit d'expression L2-bêta gal et plusieurs bandes secondaires.

## Immunogénicité du produit L2-bêta gal.

Pour tester l'immunogénicité de la protéine de fusion, le produit élué a été injecté dans deux cobayes. Les sérums ont été recueillis après la troisième injection. Par une analyse au "Western Blot", les sérums obtenus ont réagi avec des bandes migrant comme la protéine de fusion L2-bêta gal ou comme le produit L2 dans les lysats bactériens issus de cultures induites à la chaleur contenant respectivement le plasmide pHPL2-bêta gal 116 ou le plasmide pHPL2.

Dans les deux types de lysats de cellules, les sérums ont reconnu des protéines (environ 60 et 55 Kd) trouvées également dans les lysats de contrôle CAG 1139. Au moins l'une d'entre elles paraît correspondre à la protéine copurifiée avec la protéine de fusion L2-bêta gal et le bêta galactosidase.

Les sérums ont reconnu des protéines d'environ 80 Kd dans une préparation virale HPVI.a dissociée (traitée par un détergent) et dans un extrait de verrue induite par HPVI.a.

Dans un test d'immunodiffusion, les sérums de cobaye obtenus n'ont pas précipité les particules HPVI.a virales intactes utilisées comme antigènes, ce qui indiquait de nouveau que les antigènes viraux reconnus par le sérum dans la technique d'immunotransfert peuvent ne pas être disponibles à la surface du virion ou que les sérums ne précipitent pas.

Dans des sections de verrue HPVI.a congelées qui sont connues pour conserver la structure conformationnelle native des virions et des polypeptides viraux, les sérums induits par les produits d'expression de L2 n'ont pas réagi. Ce résultat témoigne encore une fois de ce que les anticorps ne sont pas dirigés contre un antigène conformationnel.

Les sérums n'ont que peu réagi (à une dilution au 1/50ème) avec des coupes de verrues infectées avec HPVI.a fixées dans le milieu de BOUIN. Ce résultat confirme le caractère spécifique de type des antigènes concernés par opposition aux antigènes de groupe qui conduisent à des réactions antigène-anticorps plus fortes avec des coupes infectées par un virus et fixées par le milieu de BOUIN.

Au contraire, les observations faites sur des coupes par le milieu de CARNOY se sont révélées fournir des réactions positives tant avec les antigènes de type qu'avec les antigènes de groupe. En particulier, on constate que les sérums de cobayes donnaient des réactions antigène-anticorps très fortes (jusqu'à une dilution de 1/1000) lorsque la coupe contenait un virus du même type que celui ayant fourni l'antigène à partir duquel avait été formé l'anticorps utilisé dans l'essai. Au contraire, aucune réaction immunologique croisée n'a été reconnue lorsque le sérum à l'étude était mis au contact de coupes contenant un virus appartenant à un type différent.

Il faut aussi préciser que les types envisagés pour les antigènes ne coïncident pas nécessairement toujours avec les types tels qu'ils ont été définis plus haut vis-à-vis des capacités d'hybridation mutuelles des génomes de deux papillomavirus distincts.

On rappellera en particulier la distinction de deux sortes d'antigènes chez les papillomavirus :
– les antigènes de type, caractérisés par l'absence de réaction antigénique croisée entre les différentes sortes de papillomavirus, en utilisant des anticorps obtenus après injection de virions entiers,
– et les antigènes de groupe, essentiellement masqués dans le virion, et mis en évidence par des anticorps après injection de particules virales dissociées.

Les papillomavirus appartenant à des types différents dans les essais de réactions anticorps-antigènes sont ceux dont les séquences codantes L2, de préférence dépourvues de leurs régions homologues respectives, codent pour des polypeptides qui ne permettent pas la réalisation de réactions antigènes-anticorps croisées avec leurs anticorps respectifs.

Des antigènes de types préférés sont ceux qui sont codés par les cadres de lecture ouverte du gène L2 dépourvu de la région N terminale, notamment sur le 1/4 de la longueur des régions L2 en cause.

L'invention concerne finalement également un test pour rechercher, dans des virus appartenant à des types différents, ceux des polypeptides codés par les régions L2 qui ne donnent pas les réactions croisées. Ces polypeptides peuvent être définis comme ceux dont les anticorps réagissent efficacement avec des virus contenus dans des coupes histologiques ou cytologiques fixées par le milieu de Carnoy, mais qui ne réagissent que mal, voire même pas du tout, avec les mêmes coupes lorsque celles-ci sont fixées par le milieu de Bouin.

Enfin, l'invention concerne aussi le procédé de diagnostic comprenant l'identification du type de virus infectieux éventuellement présent chez un patient, consistant à faire réagir des antigènes préalablement obtenus contre des virus appartenant à différents types avec un prélèvement biologique, notamment de sérum, provenant d'un malade pour qui le test doit être réalisé.

Le virus infectieux sera présumé appartenir à un type déterminé dès lors que l'on observera une réaction antigène-anticorps entre un prélèvement de sérum et un antigène provenant d'un papillomavirus appartenant à ce même type. Ces essais de diagnostic peuvent être réalisés par exemple en mettant en oeuvre la méthode d'ELISA.

Plus particulièrement l'invention concerne un procédé de détection de l'appartenance ou non d'un papillomavirus infectieux dans un échantillon biologique humain, tel qu'un tissu ou fluide, par exemple un sérum, à un type donné de papillomavirus. Ce procédé est caractérisé par la mise en contact de cet échantillon biologique avec des anticorps préalablement formés contre un produit d'expression d'un ADN contenant au moins une partie de la région L2 du génome d'un virus appartenant à ce type, cette mise en contact étant réalisée dans des conditions et pendant une durée permettant la réalisation d'une réaction immunologique. La détection de la formation d'un complexe antigène-anticorps témoigne alors de la présence d'un papillomavirus ayant un type identique ou apparenté à celui dont provenait le susdit produit d'expression.

De préférence, l'ADN contenant la région L2 ou la partie correspondante a été cloné dans un hôte cellulaire compétent, tel qu'une bactérie, par exemple E. coli.

Avantageusement la partie de région L2 mise en oeuvre correspond au cadre de lecture ouverte du gène L2 dépourvu de la région N-terminale non caractéristique de type. De préférence, cette région N-terminale correspond au premier quart de ce cadre de lecture.

Plus particulièrement, l'invention concerne un procédé de détection de ce type dans lequel l'ADN contenant la susdite partie de région L2 (ou la région L2 entière) est un ADN hybride formé à partir d'un acide nucléique codant pour une protéine normalement exprimable dans l'hôte cellulaire compétent choisi et dans lequel la susdite région L2 aura été incorporée au préalable, notamment par recombinaison in vitro.

Par exemple la protéine normalement exprimable dans l'hôte cellulaire correspond à tout ou partie de la bêta-galactosidase, lorsque cet hôte cellulaire est E. coli.

Le procédé de détection selon l'invention est applicable à toute coupe histologique fixée, de préférence dans le milieu de Carnoy, ou encore directement à un sérum.

Le procédé selon l'invention comprend également la mise en contact de l'échantillon biologique dans les conditions sus-indiquées avec des "cocktails" d'anticorps préalablement formés contre des produits d'expression des régions L2 -ou de leurs parties correspondantes respectives- originaires de plusieurs virus de mêmes types ou de types apparentés, en particulier de virus regroupés de la même façon qu'indiqué ci-dessus, en

EP 0 235 187 B1

rapport avec les sondes d'ADN.

L'utilisation de ces "cocktails" d'anticorps permet alors, dans des conditions semblables à celles qui ont été évoquées en rapport avec les sondes d'ADN, de procéder à des corrélations entre l'appartenance détectée à une catégorie donnée de papillomavirus et la maladie dont le patient étudié souffre -ou à laquelle il est potentiellement exposé.

L'invention concerne finalement un procédé de fabrication de chacun des susdits polypeptides hybrides et des anticorps correspondants.

Ce procédé comprend :

– l'incorporation, notamment in vitro, de la région L2 du génome du papillomavirus concerné ou de la partie de région correspondant dans un vecteur approprié ;

– la transformation d'un hôte cellulaire compétent avec le vecteur ainsi modifié, c'est-à-dire d'un hôte sus-cescceptible d'exprimer la susdite rétion L ou la partie corespondante ; et

– la récupération et, de préférence, la séparation du polypeptide résultant de l'expression de la région L2 ou de la partie correspondante, ce polypeptide étant capable d'induire in vivo la production d'anticorps capables de détecter des protéines du papillomavirus dans les conditions sus-indiquées.

L'invention concerne encore le procédé de production desdits anticorps, caractérisé par l'immunisation d'un animal, par exemple un lapin, avec les susdits polypeptides, et la récupération des anticorps formés.

L'invention comprend éventuellement le regroupement soit des peptides hybrides, soit des anticorps, obtenus à partir de divers papillomavirus, selon les différents types auxquels ceux-ci peuvent s'avérer appartenir.

Enfin l'invention concerne également un procédé de classification de papillomavirus connus. Ce procédé est caractérisé en ce que, après hybridation dans des conditions non strictes de son génome avec la région L2 d'un ou de plusieurs génomes de papillomavirus connus ou, le cas échéant, séquençage de son génome, et, par conséquent, repérage du cadre de lecture ouverte correspondant au gène L2, on produit un recombinant entre un fragment consistant en tout ou partie de ce gène L2 préalablement excisé dudit génome et d'un vecteur approprié, puis on induit l'expression dudit fragment dans un hôte cellulaire ou microorganisme approprié, notamment E. coli, préalablement transformé par le vecteur modifié obtenu et, le cas échéant, on produit ensuite in vivo des anticorps contre les produits d'expression obtenus, ce procédé de classification comportant des essais de réaction croisée antigène-anticorps

– soit entre les produits d'expression du gène L2 du papillomavirus nouvellement isolé et les compositions d'anticorps de l'ensemble d'anticorps, tel qu'il a été défini plus haut,

– soit entre les anticorps formés contre les produits d'expression du gène L2 du papillomavirus nouvellement isolé et les compositions de polypeptides de l'ensemble de polypeptides, tel qu'il a été défini plus haut et l'on distingue ou non le type de papillomavirus nouvellement isolé de chacun des papillomavirus correspondant à ceux des différentes compositions de l'ensemble selon le cas, soit de l'ensemble d'anticorps, soit de l'ensemble de polypeptides, tels qu'ils ont été définis plus haut, selon que les réactions antigènes-anticorps croisées conduisent à des résultats négatifs ou positifs.

## BIBLIOGRAPHIE

(1) Derst, M. et al., 1983, Proc. Natl. Acad. Sci. U.S.A., 80, 3812-3815.

(2) Coggin, J.R., Jr. et al., 1979, Cancer Res., 39, 545-546.

(3) Gissmann, L. et al., 1982, J. Virol. 44, 393-400.

(4) Green, M. et al., 1982, Proc. Natl. Acad. Sci. U.S.A. 79, 4437-4441.

(5) Heilman, C.A. et al., 1980, Virol. 36, 395-407.

(6) Jablonska, S. et al., 1972, Cancer Res., 32, 583-589.

(7) Jablonska, S. et al., 1982, Springer Semin. Immunopathol. 5, 33-62.

(8) Kremsdorf, D. et al., 1982, J. Virol. 43, 436-447.

(9) Kremsdorf, D. et al., 1983, J. Virol. 48,.340-351.

(10) Lutzner, M.A. et al., 1978, Bull. Cancer, 65, 169-182.

(11) Lutzner, M.A. et al., 1983, Lancet ii, 422-424.

(12) Migozzi, M. et al., 1965, Bull. Soc. Franc. Derm. Syph. 72, 747-748.

(13) Orth, G. et al., 1980, Cold Spring Harbor Conf. Cell Proliferation, 7, 259-282.

(14) Orth, G. et al., 1981, J. Invest. Dermatol. 76, 97-102.

(15) Orth, G. et al., 1979, Cancer Res. 39, 1074-1082.

(16) Ostrow, R.S. et al., 1982, Proc. Natl. Acad. Sci. U.S.A. 79, 1634-1638.

(17) Ostrow, R.S. et al., 1983, Ann. Acad. Dermatol. 8, 398-404.

(18) Pfister, H. et al., 1983, Cancer Res. 43, 1436-1441.

(19) Pfister, H. et al., 1983, J. Virol. 47, 363-366.

(20) Pfister, H. et al., 1981, Int. J. Cancer, 27, 645-650.

(21) Rueda, L.A. et al., 1976, Med. Cut. I.L.A. 2, 113-136.

(22) Ruiter, M. et al. J. Invest. Dermatol., 47, 247-252.

(23) Sutcliffe, J.G., 1978, Nucleic Acids Res. 5, 2721-2728.

(24) Tsumori, T. et al., 1983, J. Gen. Virol. 64, 967-969.

## Revendications

1. Ensemble de compositions, dont chacune est formée d'un groupe comprenant au moins un polypeptide, chaque polypeptide correspondant au produit d'expression de tout ou partie d'un gène E6, E7 ou, de préférence, L2, d'un papillomavirus, ces groupes étant respectivement dérivés des papillomavirus suivants :

1) HPV 2d (C.N.C.M. N° I-379) ;

2) HPV 10b (C.N.C.M. N° I-380 et N° I-381), HPV 28 (C.N.C.M. N° I-395) ;

3) HPV 24 (C.N.C.M. N° I-392 et I-393) ;

4) HPV 14 (C.N.C.M. N° I-382 et I-383), HPV 15 (C.N.C.M. N° I-384), HPV 17 (C.N.C.M. N° I-385 et N° I-386), HPV 19 (C.N.C.M. N° I-387), HPV 20 (C.N.C.M. N° I-388), HPV 21 (C.N.C.M. N° I-389), HPV 22 (C.N.C.M N° I-390) et HPV 23 (C.N.C.M. I-391) ;

5) HPV 15 (C.N.C.M. N° I-384), HPV 17 (C.N.C.M. N° I-385 et N° I-386) ;

6) HPV 24 (C.N.C.M. N° I-392 et N° I-393) ;

7) HPV 14 (C.N.C.M. N° I-382 et N° I-383), HPV 32 (C.N.C.M. N° I-397) et HPV IP4 (C.N.C.M. N° I-449) ;

8) HPV 31 (C.N.C.M. N° I-396) ;

9) HPV 32 (C.N.C.M. N° I-397) ;

10) HPV 16, HPV 18 et HPV IP2 (C.N.C.M. N° I-450), étant entendu que les polypeptides des dix compositions sont respectivement choisis de façon à être en toutes circonstances différents les uns des autres, dans la mesure où chacune des dix compositions serait réduite à un polypeptide dérivé de l'un seulement des HPVS du groupe correspondant.

2. Ensemble selon la revendication 1, caractérisé en ce que lesdites compositions contiennent respectivement, en association avec lesdits polypeptides, un véhicule pharmaceutiquement acceptable, permettant l'utilisation d'une ou de plusieurs de ces compositions pour une vaccination contre les papillomavirus correspondants.

3. Ensemble de compositions d'anticorps, dont chacune est formée d'un groupe comprenant au moins un anticorps lui-même dirigé contre un produit d'expression de tout ou partie d'un gène E6, E7, ou, de préférence, L2, d'un papillomavirus, ces groupes étant respectivement dérivés des papillomavirus suivants :

1) HPV 2d (C.N.C.M. N° I-379) ;

2) HPV 10b (C.N.C.M. N° I-380 et N° I-381), HPV 28 (C.N.C.M. N° I-395) ;

3) HPV 24 (C.N.C.M. N° I-392 et I-393) ;

4) HPV 14 (C.N.C.M. N° I-382 et I-383), HPV 15 (C.N.C.M. N° I-384), HPV 17 (C.N.C.M. N° I-385 et N° I-386), HPV 19 (C.N.C.M. N° I-387), HPV 20 (C.N.C.M. N° I-388), HPV 21 (C.N.C.M. N° I-389), HPV 22 (C.N.C.M N° I-390) et HPV 23 (C.N.C.M. I-391) ;

5) HPV 15 (C.N.C.M. N° I-384), HPV 17 (C.N.C.M. N° I-385 et N° I-386) ;

6) HPV 24 (C.N.C.M. N° I-392 et N° I-393) ;

7) HPV 14 (C.N.C.M. N° I-382 et N° I-383), HPV 32 (C.N.C.M. N° I-397) et HPV IP4 (C.N.C.M. N° I-449);

8) HPV 31 (C.N.C.M. N° I-396) ;

9) HPV 32 (C.N.C.M. N° I-397) ;

10) HPV 16, HPV 18 et HPV 1P2 (C.N.C.M. N° I-450), étant entendu que les anticorps des dix compositions sont respectivement choisis de façon à être en toutes circonstances différents les uns des autres, dans la mesure où chacune des dix compositions serait réduite à un anticorps dérivé de l'un seulement des anticorps du groupe correspondant.

4. Ensemble selon la revendication 3, caractérisé en ce que lesdites compositions contiennent respectivement en association avec lesdits anticorps un véhicule pharmaceutiquement acceptable, permettant l'utilisation d'une ou de plusieurs de ces compositions, pour induire, à l'occasion de leur administration à un sujet porteur des papillomavirus correspondants, une régression des infections induites par ces papillomavirus.

5. Procédé de diagnostic in vitro de la nature d'un papillomavirus contenu dans un prélèvement histologique ou cytologique d'un patient ou isolable à partir de ce prélèvement, caractérisé par la mise en contact de ce prélèvement avec les différents anticorps ou groupes d'anticorps de l'ensemble selon la revendication 3, et la détection de celui ou de ceux des anticorps ou groupes d'anticorps qui restent fixés à ce prélèvement.

6. Procédé selon la revendication 5, caractérisé en ce que le prélèvement se présente sous forme de préparation ou coupe histologique fixée.

7. Procédé selon la revendication 6, caractérisé en ce que la coupe histologique est fixée par le milieu de Carnoy.

8. Procédé de classification d'un papillomavirus nouvellement isolé, caractérisé en ce que, après hybridation dans des conditions non strictes de son génome avec la région L2 d'un ou de plusieurs génomes de papillomavirus connus ou, le cas échéant, séquençage de son génome, et, par conséquent, repérage du cadre de lecture ouverte correspondant au gène L2, on produit un recombinant entre un fragment consistant en tout ou partie de ce gène L2 préalablement excisé dudit génome et d'un vecteur approprié, puis on induit l'expression dudit fragment dans un hôte cellulaire ou microorganisme approprié, notamment E. coli, préalablement transformé par le vecteur modifié obtenu et, le cas échéant, on produit ensuite in vivo des anticorps contre les produits d'expression obtenus, ce procédé de classification comportant des essais de réaction croisée antigène-anticorps

– soit entre les produits d'expression du gène L2 du papillomavirus nouvellement isolé et les compositions d'anticorps de l'ensemble selon la revendication 3,

– soit entre les anticorps formés contre les produits d'expression du gène L2 du papillomavirus nouvellement isolé et les compositions de polypeptides de l'ensemble selon la revendication 1

et l'on distingue ou non le type de papillomavirus nouvellement isolé de chacun des papillomavirus correspondant à ceux des différentes compositions de l'ensemble conforme, selon le cas, à la revendication 1 ou à la revendication 3, selon que les réactions antigènes-anticorps croisées conduisent à des résultats négatifs ou positifs.

## Patentansprüche

1. Ensemble von Zusammensetzungen, von denen jede aus einer mindestens ein Polypeptid enthaltenden Gruppe gebildet ist, wobei jedes Polypeptid dem Expressionsprodukt der Gesamtheit oder eines Teils des Gens E6, E7 oder, bevorzugt, L2 eines Papillomavirus entspricht und wobei diese Gruppen jeweils von den folgenden Papillomaviren abgeleitet sind:

1) HPV 2d (C.N.C.M. Nr. I-379);

2) HPV 10b (C.N.C.M. Nr. I-380 und Nr. I-381), HPV 28 (C.N.C.M. Nr. I-395);

3) HPV 24 (C.N.C.M. Nr. I-392 und I-393);

4) HPV 14 (C.N.C.M. Nr. I-382 und I-383), HPV 15 (C.N.C.M. Nr. I-384), HPV 17 (C.N.C.M. Nr. I-385 und Nr. I-386), HPV 19 (C.N.C.M. Nr. I-387), HPV 20 (C.N.C.M. Nr. I-388), HPV 21 (C.N.C.M. Nr. I-389), HPV 22 (C.N.C.M. Nr. I-390) und HPV 23 (C.N.C.M. Nr. I-391);

5) HPV 15 (C.N.C.M. Nr. I-384), HPV 17 (C.N.C.M. Nr. I-385) und Nr. I-386);

6) HPV 24 (C.N.C.M. Nr. I-392 und Nr. I-393);

7) HPV 14 (C.N.C.M. Nr. I-382 und Nr. I-383), HPV 32 (C.N.C.M. Nr. I-397) und HPV IP4 (C.N.C.M. Nr. I-449);

8) HPV 31 (C.N.C.M. Nr. I-396);

9) HPV 32 (C.N.C.M. Nr. I-397);

10) HPV 16, HPV 18 und HPV IP2 (C.N.C.M. Nr. I-450), wobei vorausgesetzt ist, daß die Polypeptide der zehn Zusammensetzungen jeweils so ausgewählt sind, daß sie sich unter allen Umständen von den jeweils anderen unterscheiden, soweit jede der zehn Zusammensetzungen auf ein Polypeptid beschränkt , das nur von einem der HPV's der entsprechenden Gruppe abgeleitet.

2. Ensemble nach Anspruch 1, dadurch gekennzeichnet, daß die genannten Zusammensetzungen jeweils zusammen mit den genannten Polypeptiden einen pharmazeutisch verträglichen Träger enthalten, der den Einsatz einer Zusammensetzung oder mehrerer für eine Vakzination gegen die entsprechenden Papillomaviren gestattet.

3. Ensemble von Antikörperzusammensetzungen, von denen jede aus einer mindestens einen Antikörper enthaltenden Gruppe gebildet ist, der selbst gegen ein Expressionsprodukt der Gesamtheit oder eines Teils des Gens E6, E7 oder, bevorzugt, L2 eines Papillomavirus gerichtet ist, wobei diese Gruppen jeweils von den folgenden Papillomaviren abgeleitet sind:

1) HPV 2d (C.N.C.M. Nr. I-379);

2) HPV 10b (C.N.C.M. Nr. I-380 und Nr. I-381), HPV 28 (C.N.C.M. Nr. I-395);

3) HPV 24 (C.N.C.M. Nr. I-392 und I-393);

4) HPV 14 (C.N.C.M. Nr. I-382 und I-383), HPV 15 (C.N.C.M. Nr. I-384), HPV 17 (C.N.C.M. Nr. I-385 und Nr. I-386), HPV 19 (C.N.C.M. Nr. I-387), HPV 20 (C.N.C.M. Nr. I-388), HPV 21 (C.N.C.M. Nr. I-389), HPV 22 (C.N.C.M. Nr. I-390) und HPV 23 (C.N.C.M. Nr. I-391);

5) HPV 15 (C.N.C.M. Nr. I-384), HPV 17 (C.N.C.M. Nr. I-385) und Nr. I-386);

6) HPV 24 (C.N.C.M. Nr. I-392 und Nr. I-393);

7) HPV 14 (C.N.C.M. Nr. I-382 und Nr. I-383 (C.N.C.M. Nr. I-397) und HPV IP4 (C.N.C.M. Nr. I-449);

8) HPV 31 (C.N.C.M. Nr. I-396);

9) HPV 32 (C.N.C.M. Nr. I-397);

10) HPV 16, HPV 18 und HPV IP2 (C.N.C.M. Nr. I-450), wobei vorausgesetzt ist, daß die Antikörper der zehn Zusammensetzungen jeweils so ausgewählt sind, daß sie sich unter allen Umständen von den jeweils anderen unterscheiden, soweit jede der zehn Zusammensetzungen auf einen Antikörper beschränkt ist, der nur von einem der Antikörper der entsprechenden Gruppe abgeleitet ist.

4. Ensemble nach Anspruch 3, dadurch gekennzeichnet, daß die genannten Zusammensetzungen jeweils zusammen mit den genannten Antikörpern einen pharmazeutisch verträglichen Träger enthalten, der den Einsatz einer dieser Zusammensetzungen oder mehrerer zur Induktion einer Regression der von Papillomaviren induzierten Infektionen bei ihrer Verabreichung an einen Träger von entsprechenden Papillomaviren gestattet.

5. Verfahren zur in-vitro-Diagnose der Natur eines Papillomavirus, das in einer histologischen oder cytologischen Probe von einem Patienten enthalten ist oder ausgehend von dieser Probe isolierbar ist, dadurch gekennzeichnet, daß man die Probe mit den verschiedenen Antikörpern oder Gruppen von Antikörpern des Ensembles gemäß Anspruch 3 in Berührung bringt und denjenigen bzw. diejenigen Antikörper oder Gruppen von Antikörpern bestimmt, die an der Probe fixiert bleiben.

6. Verfahren nach Anspruch 5, dadurch gekennzeichnet, daß die Probe in Form eines Präparats oder eines fixierten histologischen Schnittes vorliegt.

7. Verfahren nach Anspruch 6, dadurch gekennzeichnet, daß der histologische Schnitt mittels eines Carnoy-Mediums fixiert ist.

8. Verfahren zur Klassifizierung eines neu isolierten papillomavirus, dadurch gekennzeichnet, daß man nach der Hybridisierung seines Genoms unter nicht-strikten Bedingungen mit der L2-Region eines Genoms oder mehrerer von bekannten Papillomaviren oder gegebenenfalls der Sequenzierung seines Genoms und folglich der Erkennung des dem Gen L2 entsprechenden offenen Leserasters ein Rekombinant zwischen einem zuvor aus dem genannten Genom geschnittenen, aus dem gesamten Gen L2 oder einem Teil desselben bestehenden Fragment und einem geeigneten Vektor herstellt, daß man anschließend die Expression des genannten Fragments in einem geeigneten zellulären Wirt oder Mikroorganismus, insbesondere E. coli, der zuvor mit dem erhaltenen, modifizierten Vektor transformiert wurde, induziert, und, gegebenenfalls, daß man anschließend in vivo Antikörper gegen die erhaltenen Expressionsprodukte herstellt, wobei das Klassifizierungsverfahren Versuche, bezüglich der Antigen/Antikörper-Kreuzreaktion

– entweder zwischen den Expressionsprodukten des Gens L2 des neu isolierten Papillomavirus und den Antikörperzusammensetzungen des Ensembles gemäß Anspruch 3,

– oder zwischen den gegen die Expressionsprodukte des Gens L2 des neu isolierten Papillomavirus gebildeten Antikörpern und den Polypeptidzusammensetzungen des Ensembles gemäß Anspruch 1

umfaßt, und daß man den Typ des neu isolierten Papillomavirus von jedem der Papillomaviren, die denjenigen der verschiedenen Zusammensetzungen des konformen Ensembles, je nach Fallgestaltung gemäß Anspruch 1 oder Anspruch 3, entsprechen, unterscheidet oder nicht, je nachdem, ob die Antigen/Antikörper-Kreuzreaktionen zu negativen oder positiven Ergebnissen führen.

## Claims

1. A set of compositions, each of which is formed of a group comprising at least one polypeptide, each polypeptide corresponding to the expression product of all or part of a E6-, E7- or, preferably, L2-gene of a papillomavirus, said groups being respectively derived from the following papillomaviruses:

1) HPV 2d (C.N.C.M. N° I-379) ;

2) HPV 10b (C.N.C.M. N° I-380 et N° I-381), HPV 28 (C.N.C.M. N° I-395) ;

3) HPV 24 (C.N.C.M. N° I-392 et I-393) ;

4) HPV 14 (C.N.C.M. N° I-382 et I-383), HPV 15 (C.N.C.M. N° I-384), HPV 17 (C.N.C.M. N° I-385 et N° I-386), HPV 19 (C.N.C.M. N° I-387), HPV 20 (C.N.C.M. N°I-388), HPV 21 (C.N.C.M. N° I-389), HPV 22 (C.N.C.M N° I-390) et HPV 23 (C.N.C.M. I-391) ;

5) HPV 15 (C.N.C.M. N° I-384), HPV 17 (C.N.C.M. N° I-385 et N° I-386) ;

6) HPV 24 (C.N.C.M. N° I-392 et N° I-393) ;

7) HPV 14 (C.N.C.M. N° I-382 et N° I-383), HPV 32 (C.N.C.M. N° I-397) et HPV IP4 (C.N.C.M. N° I-449) ;

8) HPV 31 (C.N.C.M. N° I-396) ;

9) HPV 32 (C.N.C.M. N° I-397) ;

10) HPV 16, HPV 18 et HPV IP2 (C.N.C.M. N° I-450), it being understood that the polypeptides of the ten compositions are respectively selected such as to differ from one another under all circumstances, to the extent where each of said ten compositions would be restricted to a polypeptide derived from one only of the HPVs of the corresponding group.

2. A set according to claim 1, characterized in that said compositions respectively contain, associated with said polypeptides, a pharmaceutically acceptable vehicle, which enables one or several of said compositions to be used for a vaccination against the corresponding papillomaviruses.

3. A set of compositions of antibodies, each of which is formed of a group comprising at least one antibody directed itself against an expression product of all or part of a E6-, E7-, or, preferably, L2-gene of a papillomavirus, said groups being respectively derived from the following papillomaviruses:

1) HPV 2d (C.N.C.M. N° I-379) ;

2) HPV 10b (C.N.C.M. N° I-380 et N° I-381), HPV 28 (C.N.C.M. N° I-395) ;

3) HPV 24 (C.N.C.M. N° I-392 et I-393) ;

4) HPV 14 (C.N.C.M. N° I-382 et I-383), HPV 15 (C.N.C.M. N° I-384), HPV 17 (C.N.C.M. N° I-385 et N° I-386), HPV 19 (C.N.C.M. N° I-387), HPV 20 (C.N.C.M. N° I-388), HPV 21 (C.N.C.M. N° I-389), HPV 22 (C.N.C.M N° I-390) et HPV 23 (C.N.C.M. I-391) ;

5) HPV 15 (C.N.C.M. N° I-384), HPV 17 (C.N.C.M. N° I-385 et N° I-386) ;

6) HPV 24 (C.N.C.M. N° I-392 et N° I-393) ;

7) HPV 14 (C.N.C.M. N° I-382 et N° I-383), HPV 32 (C.N.C.M. N° I-397) et HPV IP4 (C.N.C.M. N° I-449) ;

8) HPV 31 (C.N.C.M. N° I-396) ;

9) HPV 32 (C.N.C.M. N° I-397) ;

10) HPV 16, HPV 18 et HPV IP2 (C.N.C.M. N° I-450), it being understood that the antibodies of the ten compositions are respectively selected such as to be different from one another under all circumstances, to the extent where each of the ten compositions would be restricted to an antibody derived from one only of the antibodies of the corresponding group.

4. A set according to claim 3, characterized in that said compositions contain respectively associated with said antibodies a pharmaceutically acceptable vehicle enabling one or several of said compositions to be used for inducing, when they are administered to a subject which carries corresponding papillomaviruses, a regression of the infections induced by said papillomaviruses.

5. Process of in vitro diagnosis of the nature of a papillomavirus contained in a histological or cytological sample taken from a patient or isolable from said sample, characterized by the bringing into contact of said sample with the different antibodies or groups of antibodies of the set according to claim 3, and the detection of the one or those of the antibodies or groups of antibodies which remain bound to that sample.

6. Process according to claim 5, characterized in that the sample is presented in the form of a fixed histological preparation or section.

7. Process according to claim 6, characterized in that the histological section is fixed by the Carnoy medium.

8. Process of classification of a newly isolated papillomavirus, characterized in that, after hybridization under non strict conditions of its genome with the L2 region of one or several genomes of known papillomaviruses or, possibly, sequencing of its genome, and, consequently, localization of its open-reading frame corresponding to the L2 gene, one produces a recombinant between a fragment consisting of all or part of said L2 gene previously cleaved out from said genome and of an appropriate vector, then one induces the expression of said fragment in an appropriate cellular host or microorganism, particularly E. coli, previously transformed by the modified vector obtained and, possibly, thereupon one produces in vivo antibodies against the expression products obtained, said classification process comprising antigene-antibody cross-reaction assays

– either between the expression products of the L2 gene of the newly isolated papillomavirus and the antibody compositions of the set according to claim 3,

– or between the antibodies formed against the expression products of the L2 gene of the newly isolated papillomavirus and the compositions of polypeptides of the set according to claim 1

and one either distinguishes or does not the type of the newly isolated papillomavirus from each of the papillomaviruses corresponding to those of the different compositions of the set in accordance with claim 1 or claim 3 as may be the case, depending upon whether the antigene-antibody cross-reaction, provide positive or negative results.

FIG.1,

IPV1a

BamH I
Hind III
Pst I
Pst I
Pvu II

Sac I
Hind III
Bg I II

EcoR I
Pvu II
Bg I II

Hind II—Hpa I
EcoR I

Hind II—Hpa I
Bg I II

Bg I II

Bg I

Hind III
Hind II

PV2d

EcoR I

Hind II
Hind II

Bg I I
Pvu II
Pst I

Hind II—Hpa I

Hind II

BamH I
Pvu II

Pst I
Bg I I
BamH I
Pst I
Pvu II
Pvu II
Pst I

IPV4a

0
10
20
30
40
50
60
70
80
90
100

BamH I
Hind III
Hind III
Hind II

EcoR I

Hind II

Hind III

Hind II

EcoR I
Bg I I

Hind II—Hpa I

Hind III

FIG.2.

FIG. 3

EP 0 235 187 B1

FIG.4a

FIG.4b

HPV_23
- Bam HI
- Eco R
- Hind II
- Ava L
- Hind II-Hpa I
- Bgl I
- Hind II
- Bgl II
- Pst I
- Sac I
- Hind III
- Eco RI
- Hind II
- Pst I
- Sac I
- Bgl I
- Hind II
- Pvu II
- Eco RI
- Pst I
- Bgl II
- Ava I
- Bgl II
- Pst I

HPV_22
- Sac I
- Bgl II
- Pst I
- Hind II
- Eco RI
- Hind II
- Bgl II
- Pst I
- Hind II-Hpa I
- Hind III
- Ava I
- Hind II
- Ava I
- Pst I
- Bgl II
- Hind III
- Ava I
- Pst I
- Pst I
- Ava I-Sma I
- Hind II
- Hind III
- Hind II
- Hind III

HPV_21
- Bam HI
- Pvu II
- Hind III
- Hind II
- Hind III
- Hind II_Hpa I
- Hind II
- Hind II_Hpa I
- Pst I
- Bgl I
- Pst I
- Bgl II
- Pst I
- Eco RI
- Pst I
- Bgl II
- Pst I
- Hind II
- Hind III
- Hind II_Sal I
- Bgl I
- Pvu I
- Hind II_Hpa I
- Pst I
- Pvu I
- Eco RI
- Pst I
- Hind II
- Ava I

HPV_20
- Ava I
- Pvu II
- Hind III
- Pst I
- Bam HI
- Hind II
- Hind III
- Hind II_Hpa I
- Pst I
- Hind II-Hpa I
- Pvu II
- Bgl I
- Pst I
- Pst I
- Bgl II
- Bgl II
- Pst I
- Hind II
- Bgl II
- Hind II
- Pvu II
- Bgl I
- Bam HI
- Hind II
- Hind II
- Hind II_Hpa I
- Bam HI
- Hind II_Hpa I

HPV_19
- Bam HI
- Pst I
- Pvu I
- Hind II
- Pvu II
- Pst I
- Pst I
- Pvu II
- Ava I
- Hind II
- Hind III
- Bgl II
- Hind II
- Hind III
- Pvu II
- Hind II_Hpa I
- Pvu II
- Bgl II
- Hind II
- Eco RI
- Bgl II

FIG.5

FIG.6.

FIG.7

FIG.8.

HPV_IP 2

Bgl II (0)
Hind III (3.8)
Pst I (8.6)
Kpn I (12.5)

Pvu II (22.5)
Pst I (23.8)
Pst I (25.7)
Hind II (26.7)
Hind III (29.8)
Xba I (30.2)

Kpn I (40.4)

Bgl I (47.6)
Pst I (51.4)

Pvu II (73.5)
Hind II (74.3)
Ava I - Sma I (76.9)

Hind II - Npa I (85.7)
Hind II - Npa I (89.5)

Xba I (96.2)
Bgl II (100)

**FIG.9**

HPV_IP 4

0 —
10 —
20 —
30 —
40 —
50 —
60 —
70 —
80 —
90 —
100 —

Eco RI
Pst I
Bgl I

Pst I
Kpn I
Pst I

Kpn I

Pvu I

Hinc II
Pst I
Hinc II - Hpa I

Kpn I
BamH I
Pst I
Ava I
Hinc II
Sac I
Pst I
Hind III
Pvu II
Hinc II

BamH I

**FIG.10**

FIG.11